# EUROPEAN PATENT SPECIFICATION

(11) **EP 4 090 297 B1**
(45) Date of publication and mention of the grant of the patent: **13.12.2023**
(21) Application number: 21700094.2
(22) Date of filing: 06.01.2021
(51) Int. Cl.: A61F 5/01

(54) **DEVICE FOR SUPPORTING AT LEAST ONE FOOT ARCH OF A USER**
VORRICHTUNG ZUM STÜTZEN MINDESTENS EINES FUSSGEWÖLBES EINES BENUTZERS
DISPOSITIF DE SUPPORT D'AU MOINS UNE VOÛTE PLANTAIRE D'UN UTILISATEUR

(30) Priority: 13.01.2020 NL 2024656
(43) Date of publication of application: 23.11.2022
(73) Proprietor: Nea International B.V., 6199 AB Maastricht-Airport (NL)
(72) Inventor: VOSKUILEN, Agnes Theodora Maria, 6199 AB Maastricht-Airport (NL); VAN DER VELDE, Jelle Martijn, 6199 AB Maastricht-Airport (NL); PARMENTIER, Filip Gery Agnes, 9800 Deinze (BE)
(74) Representative: Algemeen Octrooi- en Merkenbureau B.V.
(86) International application number: PCT/NL2021/050004
(87) International publication number: WO 2021/145762

(56) References cited:
- EP-A1- 3 478 227
- US-A- 5 755 679
- US-A1- 2014 245 700
- US-B2- 10 398 584

## Description

The invention relates to a device for supporting at least one foot arch of a user.

An above-described device is known, for example, from US2013/0066249. This known device comprises a forefoot strap which extends around the forefoot across the forefoot sole and the top side of the forefoot when it is fitted to a foot. Furthermore, this device comprises a working strap which can be anchored around the heel and the ends of the at least one working strap are attached to the forefoot strap. The working strap is attached to the forefoot strap by means of stitching or sewing. The document US 5 755 679 A discloses also a device for supporting at least one foot arch of a user.

Although the above-described known single-part device can be fitted by a user in a relatively simple manner, the foot arch support which can be achieved by means of the device is not optimal and relatively limited.

It is therefore an object of the present device to provide an improved device by means of which at least one foot arch of a user can be supported in a more optimum and improved way.

This object is achieved by the device according to Claim 1. Advantageous further aspects and embodiments are the subject-matter of the dependent claims.

The device for supporting at least one foot arch of a user comprises a forefoot anchor which is configured to be anchored on the foot, at least partly at the location of the distal part of the metatarsal bones (ossis metatarsi I-V). The position of the forefoot anchor on the forefoot at the location of the distal part of the metatarsal bones, that is to say situated closer to the caput ossis metatarsi I-V than to the basis ossis metatarsi I-V or even situated against or at least partly over the caput ossis metatarsi I-V, means that the forefoot anchor has to be positioned relatively close to the toes. In this way, the distance between the heel and the forefoot anchor is relatively large, which provides an improved support for the foot arch of a user, in particular for the medial foot arch of a user, especially compared to the above device known from US2013/0066249. The device furthermore comprises at least one working strap which can be anchored around the heel and ends of this at least one working strap are attached or attachable to the forefoot anchor. It is also possible to anchor the working strap near the heel, that is to say anchor it at the location of the heel bone, viewed in the length direction of the foot, for example to the medial and/or lateral side of the foot. In addition to the position of the forefoot anchor which provides improved support for a foot arch, the device can be brought from a fitting position to a working position in order to further improve this support. In the fitting position, the at least one working strap is not or virtually not tensioned, so that the device can be fitted to a foot in a relatively simple manner, wherein a device, once it has been fitted to the foot, does not offer any or only minimal support to a foot arch of a user in the fitting position. The difference between the fitting position and the working position of the device resides in the fact that, in the working position, the at least one working strap between the heel and the forefoot anchor is tensioned in such a way that the part or parts of the tensioned at least one working strap situated under the sole support at least one foot arch of a user.

Also, it is possible to provide an additional and even optionally adjustable tension in the working strap by means of the device, which cannot be provided by the above device known from US2013/0066249 after fitting, since the working strap is sewn to the forefoot strap of the known device, as a result of which the working strap cannot be tensioned additionally after the known device has been put on the foot in the foot positions intended for the purpose and/or the tension which can be produced in the working position cannot be adjusted in order to provide an individual desired tension or support of the foot.

In this document, the term foot arch may refer both to the two foot arches extending in the length direction of the foot, the medial and lateral foot arch, and the foot arch extending in the width direction of the foot, the transversal arch. In the working position, the device described in this document is very useful in preventing at least one of these foot arches from sagging in an undesirable way during a movement of the foot. That is to say, in the working position, the device is aimed at limiting, or even preventing if desired, sagging or collapsing of at least one of the foot arches (the transversal, medial and/or lateral foot arch) and/or overpronation of the foot. In the working position, the device can offer support to the transversal foot arch substantially by means of the front anchor. The medial and/or lateral foot arch can be supported substantially by means of the at least one working strap of the device by means of the device in the working position.

The device provides relief for the plantar fascia. The device may be used to treat various disorders, such as for example plantar fasciitis and/or hallux valgus. The device may also be used to support natural foot movements in order to enhance performance, for example when playing sports.

In one aspect, the at least one working strap may be provided with a first working strap section which, in the working position, extends between the forefoot anchor near the head of metatarsal bone I (caput ossis metatarsi I) and the heel bone and at least one second working strap section which, in the working position, extends between the forefoot anchor near the head of metatarsal bone V (caput ossis metatarsi V) and the heel bone. Such working strap sections bridge a relatively large portion of the length of the foot measured between the heel and the toes. In the working position, that is to say tensioned position, such relatively long working strap sections can provide a relatively large contribution to supporting a foot arch of a user. The support of the foot arch may be improved further if, in the working position, the first and the second working strap sections cross under the sole.

In a further aspect, it is desirable for the at least one working strap to be relatively inelastic. In this case, inelastic means that an average user cannot expand the working strap manually, or only to a minimal degree. In particular, the working strap is made in such a way that the forces required to bring about an elastic deformation of the at least one working strap are greater than the forces to bring about a corresponding elastic deformation in the forefoot anchor. In other words, expanding the material from which the working strap is made over a certain distance requires a greater force than expanding the material from which the forefoot anchor is made over the same distance, in which case the term material is understood to refer not only to the choice of material, but also the structure of the material, such as for example the weave of a textile product (knitted, woven or braided), by means of which properties such as strength and deformability may be determined. By means of the at least one relatively inelastic working strap, optimum support may be provided to at least one foot arch of a user in the working position of the device.

In one embodiment, the device is a brace which can be fitted over the foot, wherein, in the fitting position, ends of the at least one working strap are not connected to the forefoot anchor, whereas the ends of the at least one working strap are releasably attachable to the forefoot anchor in order to bring the device to the working position. Such a brace is user-friendly because it is relatively easy to put on, while sufficient tension can be produced for supporting the foot arch in the working position of the brace. The construction and configuration of the brace is such that the brace can be worn in combination with a shoe. The advantage is that consequently no arch support has to be used in combination with the shoe, and that the user is assured that the foot arch is supported by means of the brace even after taking off the shoe.

The brace may comprise textile connecting parts which extend between the forefoot anchor and the heel, wherein the textile connecting parts cover at least portions of the instep and/or the sole. The brace is constructed such that different elasticity zones are provided in the brace, so that the brace is, on the one hand, relatively easy to put on in the fitting position, while offering maximum support to the foot arch in the working position of the brace and also being comfortable for a user in the working position. To this end, the forces which are required to bring about an elastic deformation in the forefoot anchor are greater than the forces to bring about a corresponding elastic deformation in the textile connecting parts. In other words, the material and/or the weave of the textile connecting parts are more elastic than the material and/or the weave of the forefoot anchor, while the material and/or the weave of the forefoot anchor are more elastic than the material and/or the weave of the at least one working strap.

In the brace, the textile connecting parts may comprise first sections and second sections, wherein the forces required to bring about an elastic deformation in the second sections are greater than the forces to bring about a corresponding elastic deformation in the first sections. It is also true that the forces required to bring about an elastic deformation in the forefoot anchor are greater than the forces to bring about a corresponding elastic deformation in the second sections. By combining the first sections and the second sections to form the textile connecting parts, it is even possible to provide zones of reduced elasticity compared to surrounding or adjacent zones in the textile connecting parts in the most elastic base component of the brace, namely the textile connecting parts. Such a zone configuration facilitates, on the one hand, a comfortable support of the foot, but also offers the possibility to give the brace a certain basic form if the brace is not on the foot of a user. This basic form may make putting on the brace easier. Furthermore, it is possible to incorporate the second sections or parts thereof in the brace in such a way that these form a tactile and/or visual mark for a user for the desired configuration of the at least one working strap.

It is also possible to provide those parts of the brace coming into contact with the foot at least partly with anti-slip material. In this way, it is possible to ensure that the brace will shift less easily in the working position during use. In particular with an inner part of the forefoot anchor contacting the naked foot, it is advantageous to improve the anchoring to the foot in order to at least partly provide it with or manufacture it from an anti-slip material. Furthermore, the second sections of the textile connecting parts may be formed by first sections which are provided with an anti-slip material. The anti-slip material then ensures that the second section is less elastic than the first section. The anti-slip material may, for example, be provided as an additional inner layer on the textile connecting parts in order to form first (without anti-slip material) and second sections.

The device may furthermore be provided with a tension-control mechanism by means of which the at least one working strap can be brought from the fitting position to the working position and vice versa. In a simple variant, the tension-control mechanism may comprise a piece of string (lace) or a buckle-like construction. By means of the piece of string or the buckle construction, an end of the at least one working strap is connected or can be connected and the forefoot anchor is connected or can be connected, wherein the working strap can be brought from the fitting position to the working position by pulling the piece of string or the buckle construction and the working strap can be brought from the working position to the fitting position by loosening or releasing the piece of string or the buckle construction. Another tension-control mechanism is for example a mechanism in which the working strap can be tightened and released by means of a relatively simple action or movement by a user, for example by means of the user rotating a turning knob, by means of which mechanism the at least one working strap can be brought from the fitting position to the working position in order to support a foot arch of a user and vice versa.

In another embodiment, the device is incorporated in a shoe, the device being provided with adjustment means for adjusting the at least one working strap between the fitting position in which the shoe with the device can be put on and removed and the working position. Such a shoe which is provided with the device described in this document is relatively easy to put on due to the construction of the device in the fitting position of the device, while it can provide maximum support in the working position of the device, wherein the degree of tightening of the at least one working strap can in addition be adjusted based on the individual or the activity to be performed. The adjustment means are situated on the outer side of the shoe, so that these can be operated relatively easily by a user in order to bring the device from the fitting position to the working position and vice versa or adjusting the tension in the working strap in the working position.

The above-described aspects will be explained below by means of exemplary embodiments in combination with the figures. However, the invention is not limited to the exemplary embodiment described below. Rather, a number of variants and modifications are possible which likewise use the idea of the invention and thus fall within the scope of protection. In particular, the possibility of combining features/aspects which have only been mentioned in the description and/or illustrated in the figures with the features of the claims insofar as compatible is mentioned.

In this case, reference is made to the following figures, in which:
Fig. 1 diagrammatically shows a bottom view of a prior-art device;
Fig. 2 diagrammatically shows a bottom view of a first embodiment of a device of a foot arch;
Figs. 3a-d show different views of a second embodiment of the device;
Figs. 4a-f show different diagrammatic views of a third embodiment of the device.

In the figures, identical components are denoted by the same reference numerals.

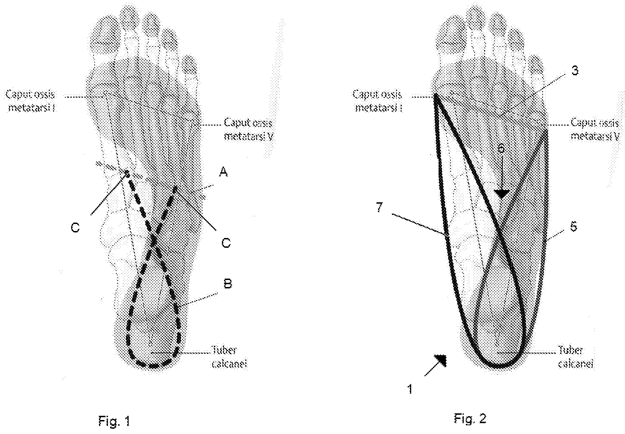

Figs. 1 and 2 diagrammatically show a basic structure of devices for supporting a foot arch and also show the anatomy of a foot with respect to the device in order to indicate the position of the devices with respect to the foot.

Fig. 1 diagrammatically shows a device known from US 2013/0066249 by means of two dashed lines A and B. Fig. 2 diagrammatically shows a first embodiment of a basic structure of the device 1.

In the device known from US 2013/0066249, an elastic forefoot strap A extends, at least over the sole, at the location of the transition between the tarsal bones (ossa tarsi) and the metatarsal bones (ossis metatarsi), that is to say on or against the basis ossis metatarsi I-V. In other words, the forefoot strap A is situated closer to the proximal part of the metatarsal bones than to the distal part of the metatarsal bones. Furthermore, the loop-shaped working strap B can be secured around the heel, with ends C of the working strap being attached on the forefoot strap B. In the fitted position, the ends C are situated under the sole of a user.

In order to clearly show the difference between the known device and the device disclosed in this document, Fig. 2 shows the basis of a first embodiment of the device 1 for supporting at least one foot arch of a user in a way similar to that of Fig. 1. The device 1 comprises a forefoot anchor 3 which is configured to be anchored on the foot, at least partly at the location of the distal part of the metatarsal bones (ossis metatarsi I-V), and two working straps 5, 7 which can be anchored around the heel and whose ends are (non-releasably) secured or (releasably) securable to the forefoot anchor 3. The fact that the forefoot anchor is situated much further in the direction of the toes of a foot in the working position than the known forefoot strap is a significant difference. As is shown in Fig. 2, the forefoot anchor 3, more particularly the part of the forefoot anchor 3 running over or along the sole, is situated closer to the caput ossis metatarsi I-V than to the basis ossis metatarsi I-V, that is to say the forefoot anchor 3 may even be situated against or at least partly over the caput ossis metatarsi I-V, which results in the forefoot anchor 3 having to be or being positioned relatively close to the toes. In this way, a relatively large distance is advantageously achieved between the forefoot anchor 3 and the heel anchor which is formed by the working straps 5, 7 for supporting at least one foot arch, as a result of which the collapsing or sagging of at least one of the foot arches (the transversal, medial and/or lateral foot arch) and/or overpronation of the foot can be limited or even prevented in an improved way.

Also, with the device 1, the attachment or the connection between the forefoot anchor 3 and the working straps 5, 7 in the working position is not secured under the sole, which may, inter alia, significantly increase comfort during use.

Figs. 2, 3a-d and 4a-f show three different embodiments of the device 1, 101, 201, wherein one of the differences between the illustrated embodiments is the way in which the working strap is or the working straps are tensioned.

In the device 1, the working straps 5, 7 are fixedly connected to the forefoot anchor 3, for example by sewing the ends of the working straps to the forefoot anchor 3 or a similar operation. Once the device 1 is fitted on the foot, in which case the forefoot anchor 3 is situated around the forefoot and/or the working straps 5, 7 are passed around the heel in order to form a heel anchor, the device 1 can be brought from a fitting position, in which the working straps are not or hardly tensioned, to a working position by a user. The device 1 can be brought from the fitting position to the working position in two ways. In the first way, the working straps 5, 7 form a heel anchor as illustrated in Fig. 2, as a result of which the device 1 is in the fitting position and by moving the forefoot anchor 3 to the desired position at the location of the distal part of the metatarsal bones, i.e. in the direction of the toes, viewed from the heel, the forefoot anchor 3 is anchored at the predetermined position by a user, as a result of which the device 1 is in the working position. In the second way, the forefoot anchor 3 is already arranged at the desired or predetermined position at the location of the distal part of the metatarsal bones, as is shown in Fig. 2, as a result of which the device 1 is in the fitting position and in this case, the device can be brought to the working position by securing the working straps 5, 7 behind the heel in order to form the heel anchor. In the working position, the working straps between the heel and the forefoot anchor are tensioned in such a way that the parts of the tensioned working straps 5, 7 situated under the sole support at least one foot arch of a user.

In the device 1, in the working position, the first working strap forms a medial loop 7 and the second working strap forms a lateral loop 5, the first and the second working straps being anchorable around the heel by means of the loop. The medial loop 7 and the lateral loop 5 both have a first section which, in the working position, partly follows the foot outline and a second section which, in the working position, extends under the foot arch, between the medial side and the lateral side. In the working position, the second sections of the loops 5, 7 cross in the crossing 6 which is situated under the sole, approximately midway between the forefoot anchor 3 and the heel anchor.

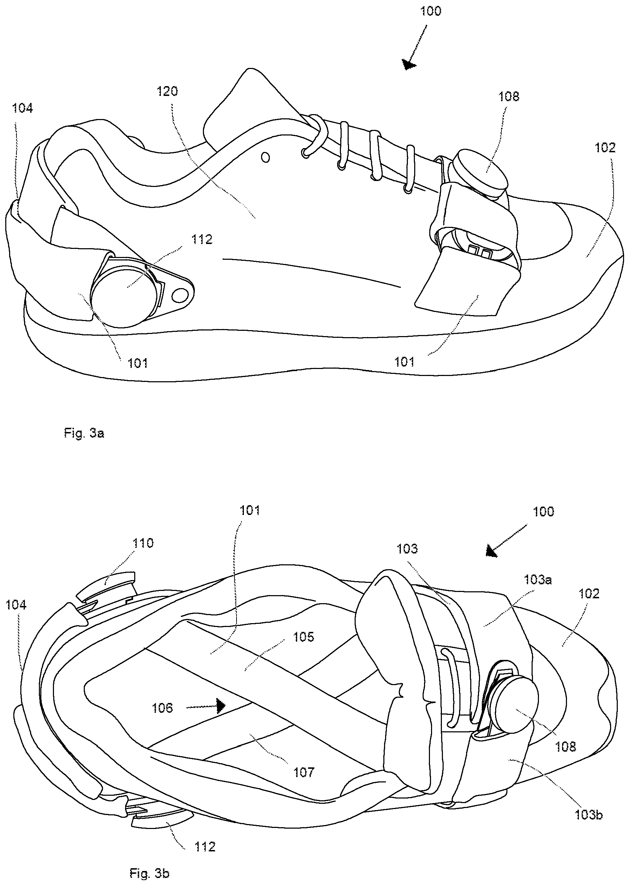

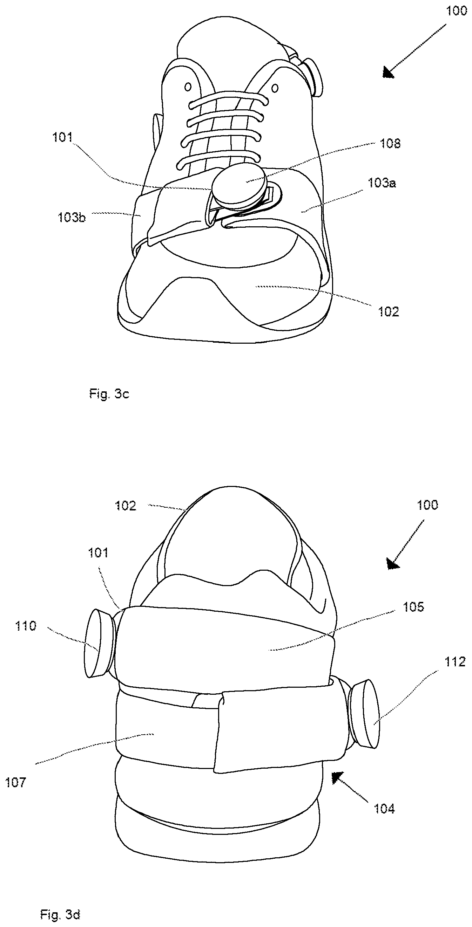

Figs. 3a-d show different views of the second embodiment of the device 101. The device 101 is incorporated in a shoe 102 which may be used to support natural foot movements so as to enhance performance, for example when playing sports or when treating a disorder of the foot, such as for example plantar fasciitis and/or hallux valgus.

The device 101 and the shoe 102 form an assembly 100. The shoe 102 is to a great extent a shoe 102 which is known per se and therefore the details thereof do not have to be described. It should however be noted that passages have been provided in the shoe, as a result of which the device 101 is situated both inside the shoe 102 and outside the shoe 102. More particularly, the passages for the working straps 105, 107 near the heel are situated on the medial and lateral sides of the shoe in order to, together with the working straps 105, 107, form the heel anchor 104 and also to form the forefoot anchor on the medial and lateral sides of the shoe. The forefoot anchor 103 is situated closer to the caput ossis metatarsi I and V than the basis ossis metatarsi I and V (as is shown in Figs. 3a-d). It is even possible to position the forefoot anchor 103 still further in the direction of the toes, for example against or at least partly over the caput ossis metatarsi (not shown). These positions of the forefoot anchor 103 and the heel anchor 104 of the device 101 which determine the relatively large distance in between achieve the same advantages as with the above-described device 1.

The device 101 basically has the same construction as the device 1. One difference is the way in which the working straps 105, 107 are tightened in order to bring the device 101 from a fitting position to a working position. Another difference is that, due to the shoe 102, the forefoot anchor 103 can be configured differently, that is to say that, due to the shoe 102, it is no longer necessary for the forefoot anchor to extend entirely around the foot (circular forefoot anchor), as a result of which the portion (not shown) of the forefoot anchor which is situated under the sole may be done away with. A circular (completely surrounding) forefoot anchor 103 is preferred in order to provide a total optimum support of the foot, for example in order to support the three abovementioned foot arches of the foot to a greater or lesser degree. Also, a further difference is the fact that the working straps 105, 107 do not comprise a loop, such as the working straps 5, 7 shown in Fig. 2. However, the working straps 105, 107 do cross at the crossing 106 under the sole between the forefoot anchor 103 and the heel anchor 104.

The forefoot anchor 103 can be anchored on the foot by means of the shoe 102, at least partly at the location of the distal part of the metatarsal bones by putting on the shoe. With a person having feet which correspond to the size of the shoe, the forefoot anchor 103 is then automatically anchored at the correct position mentioned above. The illustrated forefoot anchor 103 is provided with an adjustment means 108 by means of which the parts 103a and 103b can be tightened by moving them with respect to each other. The adjustment means 108 is optional and is advantageous when treating specific disorders or for stimulating specific movements, for example when playing sports. Furthermore, the device 101 comprises two working straps 105, 107, the first ends of which are attached to the shoe 102 via adjustment means 110, 112 of the working straps 105, 107 in order to form the heel anchor 104, and the second ends of which are attached to the forefoot anchor 103. The parts of the two working straps 105, 107 running across the heel part or rear side of the shoe 102 (see for example Fig. 3d) are not necessary and may be omitted. In this embodiment of the shoe variant (not shown), the end of each working strap is connected to one of the adjustment means, as a result of which the working straps are not anchored around the heel in order to form the heel anchor, but are anchored near the heel (at the location of the heel bone, viewed in the length direction of the foot) by means of the adjustment means (which form the heel anchor), at the lateral and medial side of the shoe, close to or on the passages in the shoe.

The device 101 can be brought from a fitting position, in which the working straps 105, 107 are not or hardly tensioned so as to be able to put the assembly 100 on the foot of the user, to a working position. In the working position, the working straps 105, 107 between the heel and the forefoot anchor 103 are tensioned/tightened by means of the adjustment means 108, 110, 112 in such a way that the parts of the tensioned working straps 105, 107 situated under the sole support at least one foot arch of a user.

The working straps 105, 107 are relatively inelastic, that is to say the forces required to bring about an elastic deformation of the working straps 105, 107 are greater than the forces required to bring about a corresponding elastic deformation of the textile material 120 of which the shoe 102 largely consists.

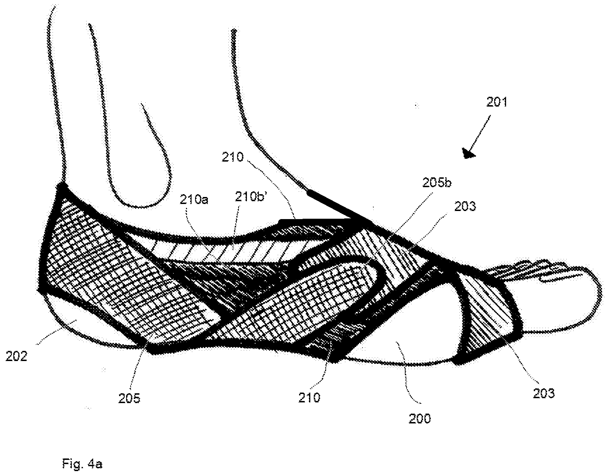

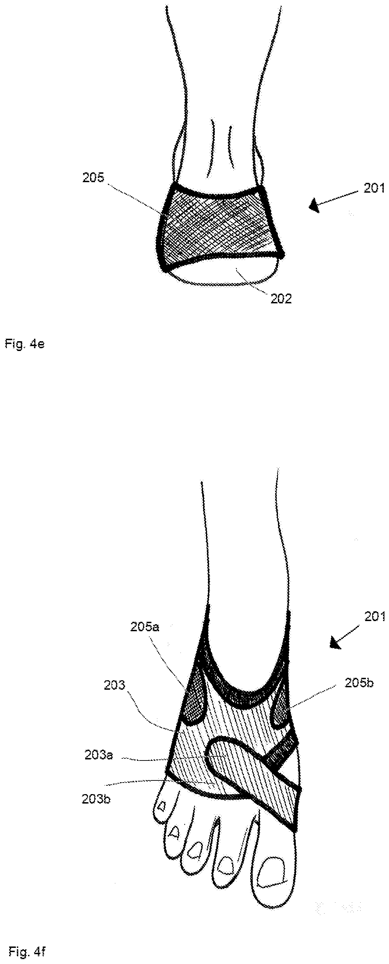

Figs. 4a-f show different diagrammatic views of a second embodiment of the device, more particularly a foot brace 201, which is attached to the foot of a user. The foot brace 201 may be used to treat various disorders, such as for example plantar fasciitis and/or hallux valgus. The foot brace 201 may also be used for supporting natural foot movements in order to enhance performance, for example when playing sports.

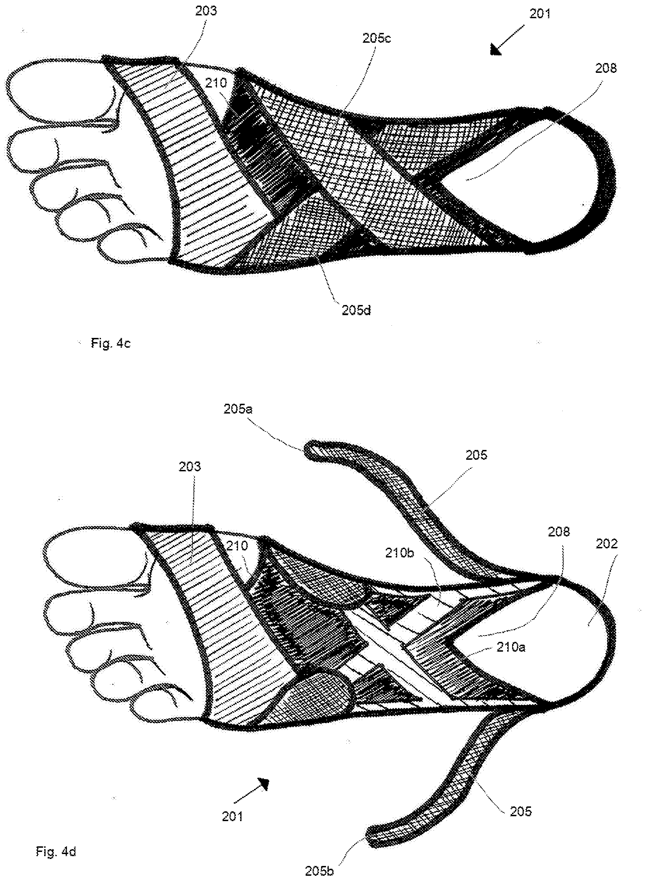

The foot brace 201 is provided with:
- a forefoot anchor 203 which is configured to be anchored on the foot 200, at least partly at the location of the distal part of the metatarsal bones (ossis metatarsi I-V), as is also shown and described with regard to the device 1, 101;
- at least one working strap 205 which can be anchored around the heel 202 and whose ends 205a, 205b are attachable to the forefoot anchor 203 by means of mechanical securing means, such as for example a hook and loop fastener, wherein the brace 201 can be brought from a fitting position, such as for example shown in Fig. 4d, in which the working strap 205 is not or hardly tensioned in order to be able to fit the device 201 to a foot of the user, to a working position (for example shown in Figs. 4a-c, and 4f), wherein, in the working position, the at least one working strap 205 is tensioned between the heel 202 and the forefoot anchor 203 in such a way that the parts of the tensioned working strap 205 situated under the sole 208 support at least one foot arch of a user. At the top side or instep side, the forefoot anchor 203 situated around the forefoot may be provided with the mechanical securing means which have already been mentioned above and cooperate with the ends 205a, 205b of the working strap 205. By releasably attaching the ends 205a, 205b of the working strap 205 on the top side or instep side to the forefoot anchor 203, both the comfort of the brace during use and the ease of use during securing and releasing of the ends 205a, 205b of the working strap 205 of the forefoot anchor 203 are increased. Also, the tension in the working strap 205 can be adjusted in a simple manner by means of the releasable attachment of the latter to the forefoot anchor 203.

As can be seen in Fig. 4c, in order to offer support, the working strap is provided with a first working strap section 205c which, in the working position, extends between the forefoot anchor near the head of metatarsal bone I (caput ossis metatarsi I) and the heel bone (calcanei) and a second working strap section 205d which, in the working position, extends between the forefoot anchor near the head of metatarsal bone V (caput ossis metatarsi V) and the heel bone. In the working position, the first and the second working strap sections 205c,d therefore cross under the sole 208. The working strap 205 may be configured as a single part, in which the ends 205a, 205b form the ends of the working strap 205. In an alternative embodiment (not shown), the working strap 205 is composed of two separate working straps which are attached to each other by means of a heel anchor section, for example by means of stitching or sewing. In use, the heel anchor section extends over the heel and is connected to the two separate working straps near the heel, on the medial and lateral side. The heel anchor section may comprise other material properties or may be made of a different material than the two separate working straps, for example, the heel section may have a certain minimum elasticity, that is to say compared to the inelastic or virtually inelastic working straps. The configuration of these two separate working straps is furthermore identical or virtually identical to the configuration of the working strap 205 as shown in Figs. 4a-f.

In order to provide, in the working position of the brace 201, a support of the at least one foot arch which has been improved still further, the forces required to bring about an elastic deformation in the working strap 205 are greater than the forces to bring about a corresponding elastic deformation in the forefoot anchor 203. In the alternative embodiment with the heel anchor section, the heel anchor section is more elastic than the two separate working straps, preferably the heel anchor-section is relatively inelastic and/or less elastic than the forefoot anchor 203.

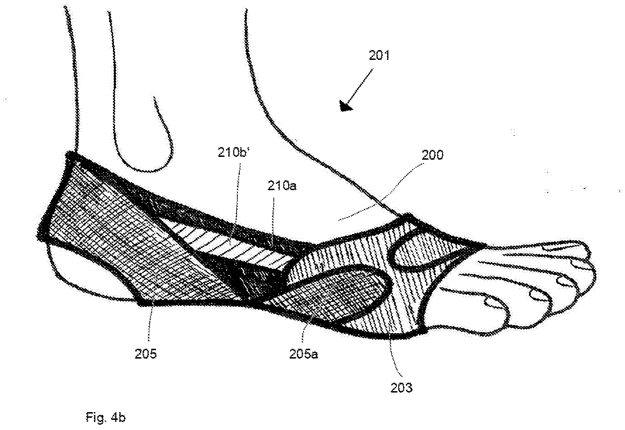

In order to improve, inter alia, the wearing comfort, the brace 201 furthermore comprises textile connecting parts 210 which extend between the forefoot anchor 203 and the heel 202 and which cover portions of the sides, the instep and/or the sole when brace 201 is being worn. The forces which are required to bring about an elastic deformation in the forefoot anchor 203 are greater than the forces to bring about a corresponding elastic deformation in the textile connecting parts 210. The textile connecting parts 210 are therefore relatively elastic. The textile connecting parts 210 of the brace may comprise first sections 210a and second sections 210b, as is shown in the figures, in particular Fig. 4d, in which case the forces which are required to bring about an elastic deformation in the second sections 210b are greater than the forces to bring about a corresponding elastic deformation in first sections 210a. The second sections 210b may optionally be provided as shown in Fig. 4d, so that these sections 210b form a tactile and/or visual mark for a user for the desired configuration of the working strap sections 205c, 205d. The edges of the brace 201 are largely or completely made of textile of the textile connecting parts 210 in order to increase the wearing comfort of a user. Although not shown in Fig. 4b, the edge of the forefoot anchor 203 on the instep of the foot 200 is preferably formed by textile of the textile connecting parts 210.

Furthermore, the foot-contacting parts of the brace 201 may, at least partly, be provided with anti-slip material. In order to improve the anchoring on the forefoot during use of the brace, a foot-contacting inner part of the forefoot anchor may, at least partly, be provided with or be made of an anti-slip material.

Furthermore, it is possible for the second sections 210b of the textile connecting parts 210 to be formed by first sections 210a on which an anti-slip material is provided, so that the advantages of a brace which remains in place during use, which may be achieved by means of the anti-slip material, can be combined with a mark for a user how the working strap sections 205c, 205d should be configured in order to put the brace 201 in the correct working position.

By combining the second sections 210b of the textile connecting parts 210 with the first sections 210a in order to form the textile connecting parts, it is possible to provide zones of reduced elasticity compared to the surrounding or adjacent zones in the textile connecting parts 210 even in the most elastic base component of the brace, namely the textile connecting parts. Such a zone configuration which may be achieved, for example, inter alia by means of the second sections 210b' (Figs. 4a, 4b) which extend over the sides of the foot, facilitates, on the one hand, a comfortable support of the foot, but also offers the possibility to give the brace a certain basic shape (not shown) if the brace is not on the foot of a user. By means of this shape, it is easier for a user to put on the brace 201 in a correct manner.

Furthermore, Fig. 4f shows that the forefoot anchor 203 may be made from two parts 203a, 203b which can be connected to each other, which parts 203a, 203b can releasably be connected to each other by means of mechanical securing means, such as for example a hook and loop fastener, in order to form a circular (entirely surrounding) forefoot anchor 203. Such a two-part configuration of the forefoot anchor 203 makes it easier to put on the brace 201 and makes advantageous use of the ball of the foot in order to bring about a relatively strong anchoring of the brace without this configuration having an effect on the support to be provided for the at least one foot arch. The circular forefoot anchor 203 may furthermore be composed of zones having different properties or consisting of different materials in order to produce a forefoot anchor having the desired elastic deformation, for example, the part 203a may be made from a different material or have a different material thickness than the part 203b of the forefoot anchor. In this way, it is possible for the forces required to bring about an elastic deformation in the part 203b to be greater than in the part 203a of the forefoot anchor 203. Obviously, it is also possible to design the forefoot anchor as a variant which is not (manually) releasable, in which case the desired anchoring is brought about by configuring the forefoot anchor in such a way that it is anchored at a predetermined position on the foot.

In the working position, the device described in this document stimulates the perception and proprioception of a user and this solely by the fact that the device is present on the body. The compression on and support of the foot on the body which the device, in particular the brace, provides, results in an increased proprioceptive perception, as a result of which a user can or will perform his/her movements in a more controlled manner. The device thus ensures that a user has more control over his/her foot/feet.

## Claims

1. Device (1; 101; 201) for supporting at least one foot arch of a user, which device is provided with:
- a forefoot anchor (3; 103; 203) which is configured to be anchored on the foot (200), at least partly at the location of the distal part of the metatarsal bones (ossis metatarsi I-V);
- at least one working strap (5, 7; 105, 107; 205) which can be anchored around the heel (202) and ends (205a, 205b) of this at least one working strap are attached or attachable to the forefoot anchor, wherein the device can be brought from a fitting position, in which the at least one working strap is not or virtually not tensioned in order to be able to put the device on a foot of the user, to a working position, in which, in the working position, the at least one working strap is tensioned between the heel and the forefoot anchor in such a way that the part or parts of the tensioned at least one working strap situated under the sole (208) between the heel and the forefoot anchor support the medial and/or lateral foot arch of a user.

2. Device (1; 101; 201) according to Claim 1, wherein the at least one working strap is provided with a first working strap section which, in the working position, extends between the forefoot anchor near the head of metatarsal bone I (caput ossis metatarsi I) and the heel bone and at least one second working strap section which, in the working position, extends between the forefoot anchor near the head of metatarsal bone V (caput ossis metatarsi V) and the heel bone, and/or the first and the second working strap sections, in the working position, cross under the sole.

3. Device (1; 101; 201) according to Claim 2, wherein, in the working position, the first working strap section is a medial loop and/or the second working strap section is a lateral loop and/or the first and/or the second working strap section are anchorable around the heel by means of the loop.

4. Device (1; 101; 201) according to Claim 3, wherein the medial loop and/or the lateral loop, in the working position, comprise a first loop section which follows the foot outline and comprise a second loop section which extends under the foot arch, between the medial side and the lateral side.

5. Device (1; 101; 201) according to one of the preceding claims, wherein the forces required to bring about an elastic deformation of the at least one working strap are greater than the forces to bring about a corresponding elastic deformation in the forefoot anchor.

6. Device (1; 201) according to one of the preceding claims, wherein the device is a brace which can be fitted over the foot, wherein, in the fitting position, ends of the at least one working strap are not connected to the forefoot anchor, whereas the ends of the at least one working strap are releasably attachable to the forefoot anchor in order to bring the device to the working position.

7. Device (1; 201) according to Claim 6, wherein the brace comprises textile connecting parts (210) which extend between the forefoot anchor and the heel and which cover at least portions of the instep and/or the sole, wherein the forces required to bring about an elastic deformation in the forefoot anchor are greater than the forces to bring about a corresponding elastic deformation in the textile connecting parts.

8. Device (1; 201) according to Claim 7, wherein the textile connecting parts comprise first sections (210a) and second sections (210b), wherein the forces required to bring about an elastic deformation in the second sections are greater than the forces to bring about a corresponding elastic deformation in first sections.

9. Device (1; 201) according to Claim 8, wherein the second sections or parts thereof are provided in such a way that these form a tactile and/or visual mark for the desired configuration of the at least one working strap.

10. Device (1; 201) according to one of Claims 6-9, wherein foot-contacting parts of the brace are, at least partly, provided with anti-slip material, preferably a foot-contacting inner part of the forefoot anchor is at least partly provided with or made of an anti-slip material.

11. Device (1; 201) according to one of Claims 8-10, wherein the second sections are formed by first sections on which an anti-slip material is provided.

12. Device (101) according to one of the preceding Claims 1-5, wherein the device is incorporated in a shoe, the device being provided with adjustment means (108) for adjusting the at least one working strap between the fitting position in which the shoe with the device can be put on and removed and the working position.

13. Device (101) according to Claim 12, wherein the adjustment means are situated on the outer side of the shoe.

14. Device (1; 101; 201) according to one of the preceding claims, wherein, in the working position, a tension in the at least one working strap can be adjusted.

15. Device (1; 101; 201) according to one of the preceding claims, wherein the device is provided with a tension-control mechanism by means of which the at least one working strap can be brought from the fitting position to the working position and vice versa.

## Patentansprüche

1. Vorrichtung (1; 101; 201) zum Stützen mindestens eines Fußgewölbes eines Benutzers, wobei die Vorrichtung bereitgestellt ist mit:
- einem Vorderfußanker (3; 103; 203), der dazu ausgelegt ist, an dem Fuß (200) verankert zu werden, mindestens teilweise an der Stelle des distalen Teils der Mittelfußknochen (Ossis Metatarsi I-V);
- mindestens einem Arbeitsriemen (5, 7; 105, 107; 205), der rund um die Ferse (202) verankert werden kann, und die Enden (205a, 205b) dieses mindestens einen Arbeitsriemens sind an dem Vorderfußanker befestigt oder befestigbar, wobei die Vorrichtung aus einer Anpassposition, in der der mindestens eine Arbeitsriemen nicht oder praktisch nicht gespannt ist, um die Vorrichtung an einen Fuß des Benutzers anbringen zu können, in eine Arbeitsposition gebracht wird, wobei, in der Arbeitsposition, der mindestens eine Arbeitsriemen zwischen der Ferse und dem Vorderfußanker derart gespannt ist, dass der Teil oder Teile des gespannten mindestens einen Arbeitsriemens, der sich zwischen der Ferse und dem Vorderfußanker unter der Sohle (208) befindet, das mediale und/oder laterale Fußgewölbe eines Benutzer stützt.

2. Vorrichtung (1; 101; 201) nach Anspruch 1, wobei der mindestens eine Arbeitsriemen mit einem ersten Arbeitsriemenabschnitt bereitgestellt ist, der sich, in der Arbeitsposition, zwischen dem Vorderfußanker nahe dem Kopf des Mittelfußknochens I (Caput ossis metatarsi I) und dem Fersenbein erstreckt und mindestens einem zweiten Arbeitsriemenabschnitt, der sich, in der Arbeitsposition, zwischen dem Vorderfußanker nahe dem Kopf des Mittelfußknochens V (Caput ossis metatarsi V) und dem Fersenbein erstreckt, und/oder dem ersten und dem zweiten Arbeitsriemenabschnitt, in der Arbeitsposition, quer unter der Sohle.

3. Vorrichtung (1; 101; 201) nach Anspruch 2, wobei, in der Arbeitsposition, der erste Arbeitsriemenabschnitt eine mediale Schlaufe ist und/oder der zweite Arbeitsriemenabschnitt eine laterale Schlaufe ist und/oder der erste und/oder der zweite Arbeitsriemenabschnitt mithilfe der Schlaufe rund um die Ferse verankerbar ist.

4. Vorrichtung (1; 101; 201) nach Anspruch 3, wobei die mediale Schlaufe und/oder die laterale Schlaufe, in der Arbeitsposition, einen ersten Schlaufenabschnitt umfasst, der dem Fußumriss folgt und einen zweiten Schlaufenabschnitt umfasst, der sich unter dem Fußgewölbe erstreckt, zwischen der medialen Seite und der lateralen Seite.

5. Vorrichtung (1; 101; 201) nach einem der vorhergehenden Ansprüche, wobei die Kräfte, die erforderlich sind, um eine elastische Verformung des mindestens einen Arbeitsriemens zu bewirken, größer sind als die Kräfte, um eine entsprechende elastische Verformung in dem Vorderfußanker zu bewirken.

6. Vorrichtung (1; 201) nach einem der vorhergehenden Ansprüche, wobei die Vorrichtung eine Klammer ist, die über dem Fuß angepasst werden kann, wobei, in der Anpassposition, die Enden des mindestens einen Arbeitsriemens nicht mit dem Vorderfußanker verbunden sind, wohingegen die Enden des mindestens einen Arbeitsriemens lösbar an dem Vorderfußanker anbringbar sind, um die Vorrichtung in die Arbeitsposition zu bringen.

7. Vorrichtung (1; 201) nach Anspruch 6, wobei die Klammer textile Verbindungsteile (210) umfasst, die sich zwischen dem Vorderfußanker und der Ferse erstrecken und die mindestens Abschnitte des Spanns und/oder der Sohle bedecken, wobei die Kräfte, die erforderlich sind, um eine elastische Verformung in dem Vorderfußanker zu bewirken, größer sind als die Kräfte, um eine entsprechende elastische Verformung in den textilen Verbindungsteilen zu bewirken.

8. Vorrichtung (1; 201) nach Anspruch 7, wobei die textilen Verbindungsteile erste Abschnitte (210a) und zweite Abschnitte (210b) umfassen, wobei die Kräfte, die erforderlich sind, um eine elastische Verformung in den zweiten Abschnitten zu bewirken, größer sind als die Kräfte, um eine entsprechende elastische Verformung in den ersten Abschnitten zu bewirken.

9. Vorrichtung (1; 201) nach Anspruch 8, wobei die zweiten Abschnitte oder Teile davon derart bereitgestellt sind, dass diese eine taktile und/oder visuelle Markierung für die gewünschte Auslegung des mindestens einen Arbeitsriemens bilden.

10. Vorrichtung (1; 201) nach einem der Ansprüche 6-9, wobei die Teile der Klammer, die mit dem Fuß in Berührung kommen, mindestens teilweise, mit einem rutschfesten Material bereitgestellt sind, vorzugsweise ist ein Teil des Vorderfußankers, der mit dem Fuß in Berührung kommt, mindestens teilweise mit einem rutschfesten Material bereitgestellt oder daraus hergestellt.

11. Vorrichtung (1; 201) nach einem der Ansprüche 8-10, wobei die zweiten Abschnitte von ersten Abschnitten gebildet werden, auf denen ein rutschfestes Material bereitgestellt ist.

12. Vorrichtung (101) nach einem der Ansprüche 1-5, wobei die Vorrichtung in einem Schuh integriert ist, wobei die Vorrichtung mit einem Einstellmittel (108) zum Einstellen des mindestens einen Arbeitsriemens zwischen der Anpassposition, in der der Schuh mit der Vorrichtung angezogen und entfernt werden kann, und der Arbeitsposition bereitgestellt ist.

13. Vorrichtung (101) nach Anspruch 12, wobei sich das Einstellmittel an der Außenseite des Schuhs befindet.

14. Vorrichtung (1; 101; 201) nach einem der vorhergehenden Ansprüche, wobei, in der Arbeitsposition, eine Spannung in dem mindestens einen Arbeitsriemen eingestellt werden kann.

15. Vorrichtung (1; 101; 201) nach einem der vorhergehenden Ansprüche, wobei die Vorrichtung mit einem Spannungssteuermechanismus bereitgestellt ist, mittels dem der mindestens eine Arbeitsriemen aus der Anpassposition in die Arbeitsposition gebracht werden kann und umgekehrt.

## Revendications

1. Dispositif (1 ; 101 ; 201) pour supporter au moins une voûte plantaire d'un utilisateur, lequel dispositif est pourvu de :
- un ancrage d'avant-pied (3 ; 103 ; 203) qui est configuré pour être ancré sur le pied (200), au moins partiellement à l'emplacement de la partie distale des os du métatarse (ossis métatarsi I-V) ;
- au moins une sangle de travail (5, 7 ; 105, 107 ; 205) qui peut être ancrée autour du talon (202) et des extrémités (205a, 205b) de cette au moins une sangle de travail sont fixées ou peuvent être fixées à l'ancrage d'avant-pied, dans lequel le dispositif peut être amené d'une position d'ajustement, dans laquelle l'au moins une sangle de travail n'est pas ou quasiment pas tendue afin de pouvoir poser le dispositif sur un pied de l'utilisateur, à une position de travail, dans laquelle, dans la position de travail, l'au moins une sangle de travail est tendue entre le talon et l'ancrage d'avant-pied de sorte que la partie ou les parties de l'au moins une sangle de travail tendue situées sous la plante (208) entre le talon et l'ancrage d'avant-pied supportent la voûte plantaire médiale et/ou latérale d'un utilisateur.

2. Dispositif (1 ; 101 ; 201) selon la revendication 1, dans lequel l'au moins une sangle de travail est pourvue d'une première section de sangle de travail qui, dans la position de travail, s'étend entre l'ancrage d'avant-pied près de la tête de l'os du métatarse I (caput ossis métatarsi I) et l'os du talon et d'au moins une deuxième section de sangle de travail qui, dans la position de travail, s'étend entre l'ancrage d'avant-pied près de la tête de l'os du métatarse V (caput ossis métatarsi V) et l'os du talon, et/ou les première et deuxième sections de sangle de travail, dans la position de travail, se croisent sous la plante.

3. Dispositif (1 ; 101 ; 201) selon la revendication 2, dans lequel, dans la position de travail, la première section de sangle de travail est une boucle médiale et/ou la deuxième section de sangle de travail est une boucle latérale et/ou la première et/ou la deuxième section de sangle de travail peut/peuvent être ancrée(s) autour du talon au moyen de la boucle.

4. Dispositif (1 ; 101 ; 201) selon la revendication 3, dans lequel la boucle médiale et/ou la boucle latérale, dans la position de travail, comprennent une première section de boucle qui suit le contour de pied et comprennent une deuxième section de boucle qui s'étend sous la voûte plantaire, entre le côté médial et le côté latéral.

5. Dispositif (1 ; 101 ; 201) selon l'une des revendications précédentes, dans lequel les forces nécessaires pour provoquer une déformation élastique de l'au moins une sangle de travail sont supérieures aux forces permettant de provoquer une déformation élastique correspondante dans l'ancrage d'avant-pied.

6. Dispositif (1 ; 201) selon l'une des revendications précédentes, dans lequel le dispositif est un appareil orthopédique qui peut être ajusté sur le pied, dans lequel, dans la position d'ajustement, les extrémités de l'au moins une sangle de travail ne sont pas reliées à l'ancrage d'avant-pied, tandis que les extrémités de l'au moins une sangle de travail peuvent être fixées de manière amovible à l'ancrage d'avant-pied afin d'amener le dispositif à la position de travail.

7. Dispositif (1 ; 201) selon la revendication 6, dans lequel l'appareil orthopédique comprend des parties de liaison textiles (210) qui s'étendent entre l'ancrage d'avant-pied et le talon et qui recouvrent au moins des portions du cou-de-pied et/ou de la plante, dans lequel les forces nécessaires pour provoquer une déformation élastique dans l'ancrage d'avant-pied sont supérieures aux forces permettant de provoquer une déformation élastique correspondante dans les parties de liaison textiles.

8. Dispositif (1 ; 201) selon la revendication 7, dans lequel les parties de liaison textiles comprennent des premières sections (210a) et des deuxièmes sections (210b), dans lequel les forces nécessaires pour provoquer une déformation élastique dans les deuxièmes sections sont supérieures aux forces permettant de provoquer une déformation élastique correspondante dans les premières sections.

9. Dispositif (1 ; 201) selon la revendication 8, dans lequel les deuxièmes sections ou des parties de celles-ci sont prévues de sorte qu'elles forment une marque tactile et/ou visuelle pour la configuration souhaitée de l'au moins une sangle de travail.

10. Dispositif (1 ; 201) selon l'une des revendications 6 à 9, dans lequel les parties de l'appareil orthopédique en contact avec le pied sont, au moins partiellement, pourvues d'un matériau antidérapant, de préférence une partie interne en contact avec le pied de l'ancrage d'avant-pied est au moins partiellement pourvue de ou réalisée en un matériau antidérapant.

11. Dispositif (1 ; 201) selon l'une des revendications 8 à 10, dans lequel les deuxièmes sections sont formées par des premières sections sur lesquelles est prévu un matériau antidérapant.

12. Dispositif (101) selon l'une des revendications précédentes 1 à 5, dans lequel le dispositif est incorporé dans une chaussure, le dispositif étant pourvu de moyens de réglage (108) pour régler l'au moins une sangle de travail entre la position d'ajustement dans laquelle la chaussure avec le dispositif peut être enfilée et enlevée et la position de travail.

13. Dispositif (101) selon la revendication 12, dans lequel les moyens de réglage sont situés sur le côté externe de la chaussure.

14. Dispositif (1 ; 101 ; 201) selon l'une des revendications précédentes, dans lequel, dans la position de travail, une tension dans l'au moins une sangle de travail peut être réglée.

15. Dispositif (1 ; 101 ; 201) selon l'une des revendications précédentes, dans lequel le dispositif est pourvu d'un mécanisme de commande de tension au moyen duquel l'au moins une sangle de travail peut être amenée de la position d'ajustement à la position de travail et vice versa.
